# EUROPEAN PATENT APPLICATION

(11) **EP 4 822 338 A1**
(43) Date of publication of application: **14.10.2026**
(21) Application number: 25315128.6
(22) Date of filing: 11.04.2025
(51) Int. Cl.: G01N 21/64, G01N 21/77, G01N 21/94, G01N 33/02, G01N 33/18

(54) **METHOD FOR DETERMINING THE PRESENCE OF CHLORDECONE IN A LIQUID SAMPLE USING FLUOROPHORE DYE-LOADED NANOEMULSIONS**

(71) Applicant: SKILLCELL, 97122 Baie Mahault (FR); Centre National de la Recherche Scientifique (CNRS), 75016 Paris (FR)
(72) Inventor: Molina, Franck, 34270 LES MATELLES (FR); Champigneux, Pierre, 34070 MONTPELLIER (FR); Klymchenko, Andrey, 67400 ILLKIRCH-GRAFFENSTADEN (FR)
(74) Representative: Yes My Patent

(57) **Abstract**

The present invention is directed to method for determining the presence of pollutants, preferably chlordecone in a liquid sample using fluorophore-loaded nanoemulsions. Finally, the invention is directed to kit comprising fluorophore-loaded nanoemulsions diluted in an aqueous phase and their uses for the determination of pollutants.

## Description

The present invention is directed to method for determining the presence of pollutants, preferably chlordecone in a liquid sample using fluorophore-loaded nano-emulsions (fluorophore-loaded NEs). Finally, the invention is directed to kit comprising fluorophore-loaded NEs diluted in an aqueous phase and their uses for the determination of pollutants.

Chlordecone (CLD), a Persistent Organic Pollutant, is still present in water and food chain of the French West Indies (FWI) (Martinique, Guadeloupe, ...), leading to dramatical public health problems.

Indeed, this organochlorine insecticide has been widely used to control banana weevil in the French West Indies. As a result of this intense use, up to 20,000 ha are contaminated by this insecticide in the French West Indies, and this causes environmental damage and health problems. A scenario of exposure was drawn by French authorities, based on land usage records. Many efforts have been made to monitor the occurrence of chlordecone and its main metabolites using different analytical methods, including GC, GC/MS, LC/MS, and NIRS. Although these different methods allow for the detection and quantification of chlordecone from soils, none of them estimates the bottle- neck caused by extraction of this organochlorine from soils with high adsorption ability (4).

Chlordecone has been synthesized and used worldwide. This molecule is composed of a bishomocubane cage, ten chlorine atoms and a ketone function which hydrates spontaneously, leading to a stable gem-diol form known as chlordecone-hydrate. Its particular structure gives it specific properties such as a strong hydrophobicity (solubility estimated around 2 mg/L in water at pH 7), a strong affinity for organic matter, and above all an acute and chronic toxicity. This pesticide was applied in the French West Indies against the banana black weevil from 1972 to 1993. Classified as Persistent Organic Pollutant in 2009, it is currently responsible for numerous environmental, socio-economic and public health problems in the FWI. Notably, it still contaminates the hydric system, soils, the food chain and consequently humans. Furthermore, even if several analytical techniques have been developed over the years to quantify and monitor chlordecone, they suffer from two major limitations: firstly, they require rather expensive devices and equipment (e.g. GC-MS/ MS and LC-MS/MS); secondly, moderate to high uncertainties remain due to the strong fixation of chlordecone on organic matter.

Thus, a convenient, fast, cheap and robust method to detect chlordecone in water and which can be directly performed on the polluted sites has still to be developed (1).

To address the issue of accurate, easy, non-expensive, sensitive and specific detection of chlordecone, a supramolecular approach could be of great interest.

Over the past few years, several research teams have developed fluorescence-based tools and synthesized organic hosts with recognition capabilities to detect pesticides. For instance, the association of cyclodextrins, calixarenes, or cucurbiturils with fluorophores has allowed the detection and quantification of pesticides, including several organochlorines sometimes in environmental matrices (5).

### Dyes and dye-loaded NEs

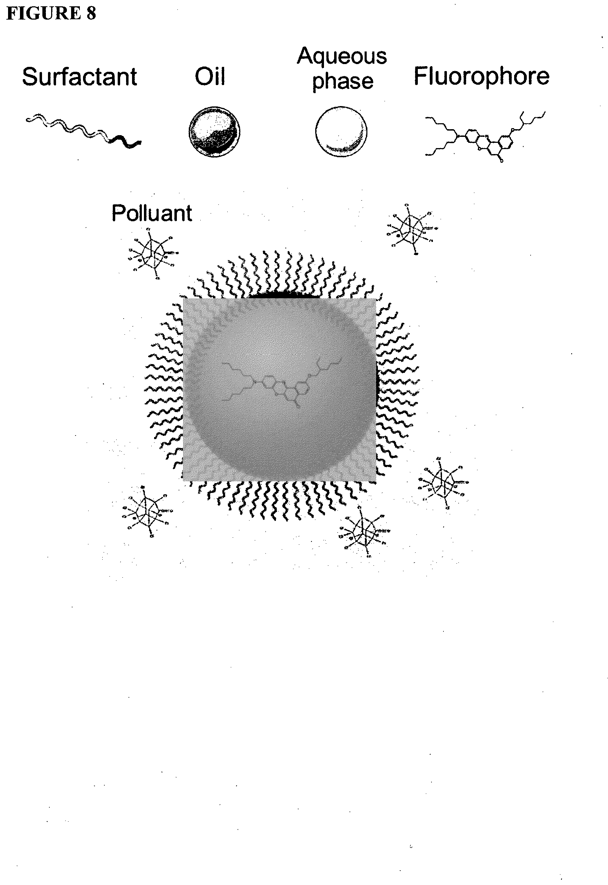

Light has been shown to be an attractive trigger for release of active molecules from nanocarriers in biological systems. For instance it has been described (2) a phenomenon of light-induced release of a fluorescent dye from lipid nano-droplets under visible light conditions. Using auto-emulsification process, nanoemulsion droplets of 32 nm size has been thus prepared encapsulating the hydrophobic analogue of Nile Red (CAS number 7385-67-3), NR668 (see A.S Klymchenko et al , 2020 (3), Figure 8A, page 12/27 ." Imaging cells incubated with NEs loaded with Nile Red and its lipophilic derivative NR668. A) Presentation of NEs loaded with Nile Red or NR668.

Fluorescent nanoemulsions (NEs) loaded with dyes is an expanding field for developing novel probes for biological and biomedical applications. It has a unique position with respect to all other existing nanoparticles, with its strong and weak points. (biocompatibility and biodegradability, can be readily prepared in largen quantities, with industrial scale production..). Actually, nanoemulsions are already frequently used as drug nanocarriers in some daily-used pharmaceutical products, such as topical products of drops or creams. Moreover, NEs constitute an important platform for future food industry. However, in the field of dye-loaded NEs a number of challenges should be addressed in order to establish them as efficient imaging agents in biology and medicine.

Although the dye-loaded nanoparticles have been intensively studied, the research on dye-loaded NEs is still in its infancy. The goal of a successful formulation of dye-loaded NEs is a high and efficient loading, high particle brightness and stability with minimal dye leakage. Indeed, the dye leakage diminishes the brightness of fluorescent NEs, but increases the background because of the leaked dyes. Dyes inside NEs are generally loaded by non-covalent methods, which means that the dye is physically entrapped inside the non-polar oil environment of NEs. In this method, dyes are dissolved in the oil prior to the process of NEs formation. In some cases, the nano-emulsification process needs to be adapted to prevent the dye degradation, for example by modulating the temperature or changing the type of surfactant. In cases when dyes have limited solubility in oil, an organic solvent is concomitantly used with dyes, surfactant and oil. It is then evaporated before the nano-emulsification, while the dye solubility in oil is improved due to the amphiphiles (surfactants) (3).

One of the major issues is the lack of an easy, non-expensive, sensitive, portable and robust method for the detection of chlordecone in aqueous phase to ensure chlordecone-free water and foods for the residents of the FWI.

This is the object of the present invention.

The present invention is directed to a non-expensive, sensitive, portable, reliable and robust method for the detection of the presence or for the quantification of organochlorines pollutants, preferably chlordecone in an environmental sample based on fluorescence shift detection of fluorophore-loaded NEs.

In the present description, the wording "fluorophore-loaded NEs" or "fluorescent dye-loaded NEs" has the same meaning.

Their simplified quantification using our lab-free approach and appropriate thresholds will faster control and ensure chlordecone-free water and foods for the residents of the FWI.

Surprisingly, the inventors have demonstrated for the first time that using particular fluorophore-loaded NEs, it is possible to detect and to determine the presence and/or the quantity of pollutants, preferably organochlorines pollutants, more preferably chlordecone, in aqueous solution.

The present inventors have demonstrated that using aqueous solution (such as aqueous buffer) containing sample to be tested, detection and quantification of the pollutant such as chlordecone can be determined, by analysing the fluorescence shift /spectra thus obtained on a standard fluorescence spectrophotometer.

Here, we propose this innovative approach based on the rapid and simple detection or quantification of pollutants such organochlorines, particularly chlordecone in aqueous solution and using particular fluorophore-loaded NEs and preparation to address the lack of advance alert tools for detecting the presence of such pesticide/insecticide toxic compounds in water, and foods.

The inventors provide experimental evidence demonstrating the technological validity, and the advantages and efficiency of this novel diagnostic approach in liquid samples

In a first aspect, the present invention is directed to a method for determining the presence and/or the quantity of pollutants in a liquid sample, said determination being based on fluorescence spectra obtained from fluorophore-loaded NEs, said method comprising the following steps of:
A) contacting said sample to be tested and susceptible to contain said pollutants with fluorophore-loaded NEs;
B) recording/capturing the fluorescence emission spectra obtained with an excitation range comprised between 520 and 536 nm, preferred is an excitation at 520 nm;
C) determining the presence and/or the quantity of pollutants by analysing the fluorescence emission spectra obtained in step B,
wherein prior to step A), said fluorophore-loaded NEs is prepared by the following steps of :
i) dissolving the fluorophores in oil and full homogenising the mixture;
ii) complementing the fluorophore-in-oil mixture with surfactant and full homogenising the mixture:
iii) adding water, preferably milli-Q^{™} /deionized water or aqueous phase, and mixing.

In a preferred embodiment, in step A), said fluorophore-loaded NEs is prior diluted in an aqueous phase (see step iii).

In step B), the excitation could range from 520 to 536 nm, and is dependent of used fluorophore. In the case of formulation of NEs with other fluorophore is used, recording protocol should be adapted.

In a preferred embodiment of the method of the present invention, said pollutants are selected from the group of organochlorine compounds, PFAS (per- and polyfluoroalkyl substances), mineral oil hydrocarbons (MOH)

In a more preferred embodiment of the method of the present invention, said pollutant is chlordecone or derived compounds thereof.

In a preferred embodiment of the method of the present invention, in step i) said fluorophore is dissolved in oil, preferably with loading of 0.5 to 5 %, in mass fraction, preferably with loading of 1% in mass fraction, heated to 80 °C, mixed at 2,000 rpm (round per minute), and vortexed until the full homogenization of the mixture.

In an also preferred embodiment of the method of the present invention, in step ii), said fluorophore-in-oil mixture obtained in step i) is complemented with surfactant to a ratio oil/surfactant comprised between 40:60 and 60:40 in mass, preferably to a ratio of 55:45 in mass.

In an also preferred embodiment of the method of the present invention, in step iii),
- the water or the aquaeous phase is added to a ratio water/oil comprised between 70:30 and 30:70, preferably to a ratio of 60:40 in mass, and
- said fluorophore-in-oil mixture obtained in step ii) is mixed for a time comprised between 5 and 15 min, preferably 10 min at room temperature.

In a more preferred embodiment of the method of the present invention, in steps i) to iii):
Oil is Labrafac^{™} CC, Fluorophore is NR668 , a lipophilic version of Nile red, the surfactant is Kolliphor^{®} ELP and the aqueous phase is Milli-Q water.

In an also preferred embodiment of the method of the present invention, the fluorophore-loaded NEs were used at a dilution comprised between 1:5 000 and 1:500 000, or comprised between 1:15 000 and 1:35 000, more preferably at dilution 1:25 000.

Said fluorophore-loaded NEs dilution can be carried out in a buffer having a pH ranging from 4 to 10, preferred are Borate, citrate, phosphate, Tris or CHES buffers, more preferred is buffer at a pH comprised between 8.0 and 9.0, preferably a borate buffer at pH 8.6

In an also preferred embodiment of the method of the present invention, the sample to be tested is mixed with the NEs 1:25 000 dilutions in equal volume 50:50 (sample:NEs) and up to 99:1 (sample:NEs) if the final dilution is always equivalent.

In another preferred embodiment of the method of the present invention, in step B) of the method of the present invention, the spectral emission datas were recorded between 350 nm and 775 nm on the IndiGo fluo (excitation LED at 520 nm), preferably the spectral emission is analysed between 540 and 700 nm.

In another preferred embodiment of the method of the present invention, in step C) the spectral characteristics was assessed by calculating the ratio R of the intensity of the two identified peaks, for example R = 1-620/1-584 or the difference X of the intensity of the two identified peaks, for example X= 1-620 - 1-584, depending of the wavelength of the two identified peaks. The ratio and/or the difference was used to determine the area of linearity of the quantification, it's LOD and LOQ once plotted against chlordecone concentration in a logarithmic scale.

In another embodiment of the method of the present invention, the sample to be tested is in presented in a liquid form, after extraction of the pollutants susceptible to be present from solid material, from soil or food.

In another aspect, the present invention relates to a method for the production of fluorophore-loaded NEs comprising the steps i), ii) and iii) as defined above in one of claims 1 to 8:
in step i) said fluorophore is dissolved in oil, preferably with loading of 0.5 to 5 %, in mass fraction, preferably with loading of 1% in mass fraction, heated to 80 °C, mixed at 2,000 rpm (round per minute), and vortexed until the full homogenization of the mixture;
in step ii), said fluorophore-in-oil mixture obtained in step i) is complemented with surfactant to a ratio oil/surfactant comprised between 40:60. and 60:40 in mass, preferably to a ratio of 55:45 in mass, in step iii);
   - the water is added to a ratio water/oil comprised between 70:30 and 30:70, preferably to a ratio of 60:40 in mass; and
   - said fluorophore-in-oil mixture obtained in step ii) is mixed for a time comprised between 5 and 15 min, preferably 10 min at room temperature, and, optionally,
the fluorophore-loaded NEs obtained were diluted at a dilution comprised between 1:15 000 and 1:35 000 dilution, more preferably at dilution 1:25 000 dilution in borate buffer at pH comprised between 8.0 and 9.0, preferably at pH 8.6.

In a preferred embodiment, the present invention relates to a method for the production of fluorophore-loaded NEs comprising the steps i), ii) and iii) as defined above wherein :
Oil is Labrafac^{™} CC, Fluorophore is NR668 , a lipophilic version of Nile red, the surfactant is Kolliphor^{®} ELP and the aqueous phase is Milli-Q water.

In another aspect, the present invention relates to a kit comprising fluorophore-in-oil mixture obtained in step iii) according to the method of production of claim 13 as described above.

Finally, in another aspect the present invention is directed to the use of a kit as described above for determining the presence or the quantity of a given pollutant susceptible to be present in a liquid sample, wherein said liquid sample to be tested is in aqueous phase.

The present invention is also directed to the use of fluorophore-in-oil mixture obtained by the process as described above in the Example for determining the presence or the quantity of a given pollutant susceptible to be present in a liquid sample, wherein said liquid sample to be tested is in aqueous phase.

The following examples, the figures and the legends hereinafter have been chosen to provide those skilled in the art with a complete description in order to be able to implement and use the present invention These examples are not intended to limit the scope of what the inventor considers to be its invention, nor are they intended to show that only the experiments hereinafter were carried out.

Other characteristics and advantages of the invention will emerge in the remainder of the description with the Examples and Figures, for which the legends are given hereinbelow.

### Figures Legends:

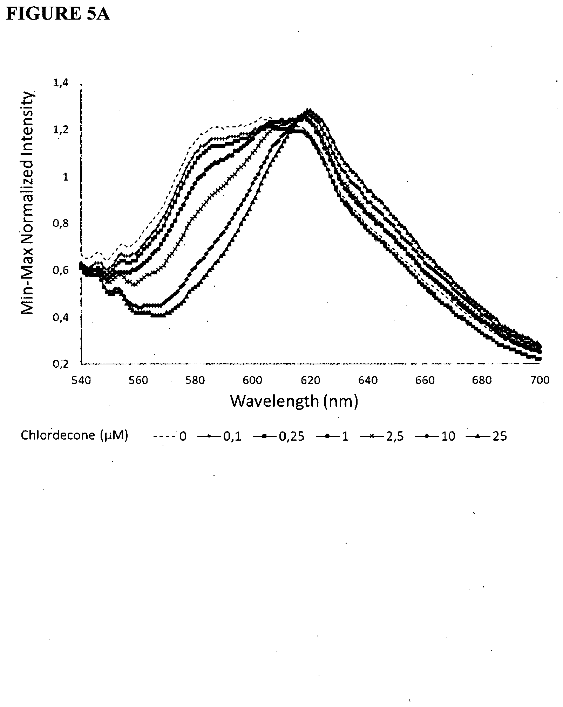

**Figure 1****:** NEs with Chlordecone (black line) and without Chlordecone dotted line)
**Figure 2****.:** NEs with HFIP (black line)) and without HFIP (dotted line))
**Figure 3****:** NEs with 1-octanol (black line), 3-Octanol (grey) and without octanol (dotted)
**Figures 4A and 4B****:**
   4A) NEs with Chlordecone (black line) and without Chlordecone dotted line).
   4B) Second derivative obtained by by Savitzky-Golay Filtering
**Figures 5A****:** Normalized mean emission spectra of fluorophore-loaded NEs in presence of increasing concentration of Chlordecone from 0.1µM up to 25 µM. No Chlordecone dotted.

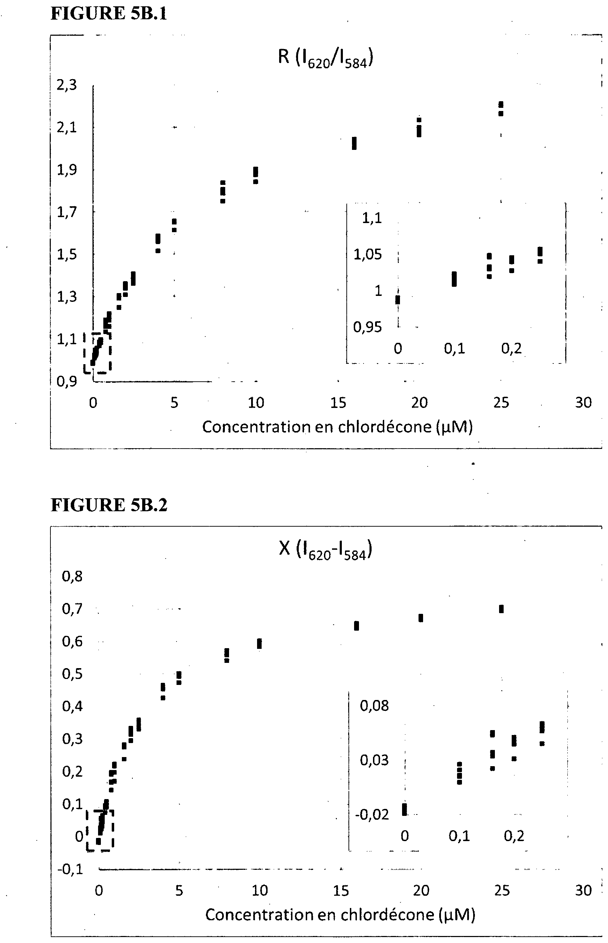

**Figures 5B.1and 5B.2**: Intensity ratio R (620 nm over 584 nm)(5B.1) and Intensity difference X (620 nm minus 584 nm)(5B.2) plotted in function of Chlordecone concentration

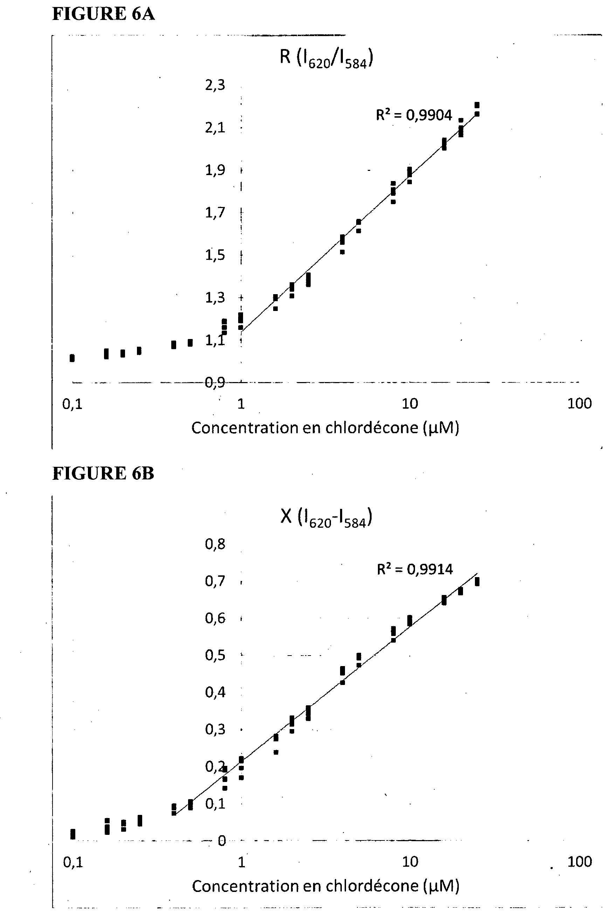

**Figures 6A and 6B** : Intensity ratio R (620 nm over 584 nm) (6A) and Intensity difference X (620 nm minus 584 nm)(6B) plotted in function of Chlordecone concentration **in logarithmic scale. The linearity domain is determined by the calculated logarithmic fit with a r²** > **0.99** It span from 1 to 25 µM of Chlordecone for the R ratio and from 0.4 to 25 µM of Chlordecone for the X difference.

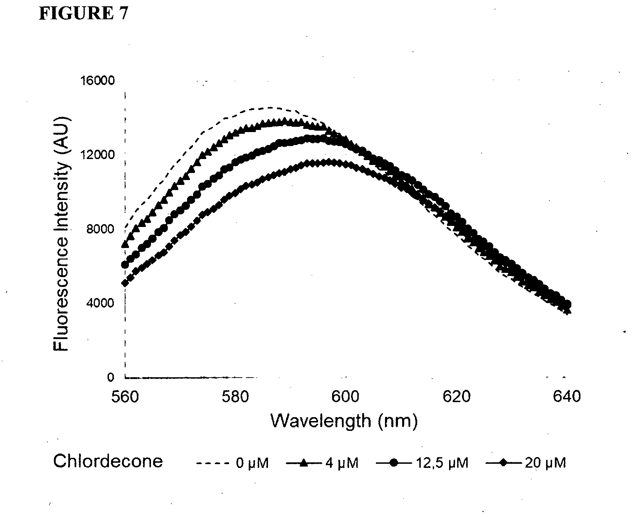

**Figure 7****:** Emission Spectra of River Water from Guadeloupe spiked with increasing concentration of chlordecone, obtained on Synergy H1 instrument
**Figure 8****:** Nano emulsion schema representing the fluorophore-loaded NEs in aqueous phase in presence of the liquid sample to be tested

### EXAMPLE 1: MATERIALS and METHODS

### A) Detection and quantification of pollutants by solvatochromic nanoemulsions

### 1) Detection principle:

The detection and quantification technology developed in this work is based on two basic concepts :
- A basic extraction technic named liquid-liquid extraction.

Liquid-liquid extraction is a valuable technique for removing hydrophobic pollutants from aqueous environments. The process leverages the low solubility of hydrophobic compounds in water and their higher affinity for hydrophobic solvent. By mixing the aqueous phase containing pollutants with an immiscible organic solvent, the hydrophobic pollutants preferentially partition into the organic phase. This method is used in environmental detection to isolate and concentrate hydrophobic contaminants, facilitating their subsequent measurements.
- A specific characteristic of some fluorescent probes named solvatochromism. Solvatochromism is an instantaneous and reversible change in spectral response of a fluorophore due to a modification of its direct physicochemical environment. This shift occurs due to differences in solvation energy between the initial state and the excited state. A more polar excited state results in a red shift (bathochromic), known as positive solvatochromism, while a less polar excited state results in a blue shift (hypsochromic), known as negative solvatochromism. The nearby concentration of a molecule such as a hydrophobic pollutant could lead to such measurable effects.

By combining these two technology to the nanometric scale, we designed nanoemulsions that can combine extraction and detection of hydrophobic pollutants from environmental samples due to solvatochromic changes of fluorophores contained in oil nano-droplets able to concentrate such pollutants.

### 2) Nano-Emulsions technology :

Nanoemulsions are colloidal systems: a liquid phase form droplets of size between 10 and 500 nm, dispersed in another immiscible liquid phase. Most common nanoemulsions are formed of oil droplets in aqueous phase. Those nanoemulsions have unique characteristics such as relative optical transparency, resistance to sedimentation or coalescence and kinetic and thermodynamic stability.

Two main methods for forming nanoemulsions exist: high-energy and low-energy methods. High-energy methods require a large amount of mechanical energy to fragment droplets in the continuous phase, thereby reducing their size to the nanoscale. These techniques involve intense fluidic forces that overcome interfacial tensions, enabling the production of nano-droplet dispersions.

In the present work, low-energy emulsification methods are used. They rely on the interaction forces of components rather than intense mechanical forces, are often more energy-efficient and allow the formation of smaller nano-droplets than high-energy methods. They primarily include two approaches: self-emulsification and phase inversion methods. Both methods can be used to synthesize our nanoemulsions.

For convenience, fluorophore-loaded NEs used in this work were prepared by spontaneous emulsification method.

### 3) Synthesis of Nano-emulsions :

Firstly, fluorophore is dissolved in oil with loading of 1% in mass fraction. The fluorophore-in-oil mixture is heated to 80 °C and mixed at 2,000 rpm, and vortexed until the full homogenization. After that, fluorophore-in-oil mixture is complemented with surfactant to a ratio oil/surfactant of 55:45 in mass. The same mixing and vortex steps are performed. Fluorophore-loaded NEs are obtained by addition of Milli-Q water to a ratio water/oil of 60:40 and after gentle mixing at room temperature for 10 min. This fluorophore-loaded NEs preparation is considered 1X concentration.

In the further results shown, the following composition is used:

| Oil | Fluorophore | Surfactant |
|---|---|---|
| Labrafac^{™} CC | NR668 - a lipophilic version of Nile red | Kolliphor^{®} ELP |
| Aqueous phase | | |
| Milli-Q water | | |

For sample fluorescence measurements, the fluorophore-loaded NEs concentration can be adapted to improve detection based on pollutants concentration. In the further experiments, the NEs were used at 1:25 000 dilutions in borate buffer (50 mM Boric Acid buffered at pH 8,6 with NaOH).

### 4) Fluorescence measurements:

Fluorescence emission spectra were captured with a plate reader BioTek Synergy H1 with an excitation of 520 nm with monochromator-based optics or a fluorescence spectrometer IndiGo fluo equipped with a 520 nm LED.

Samples are mixed with the NEs 1:25 000 dilutions in equal volume. For laboratory convenience, volumes were between 50 and 150 µL but there are not limit for higher volumes.

### EXAMPLE 2: Results

A first session of tests was performed with various pollutants at the limit of solubility to characterize the potential shift. Spectral emission datas were recorded between 350 nm and 775 nm on the IndiGo fluo (excitation LED at 520 nm). Spectra are smoothed by Gaussian smoothing of SD 1, analyzed between 540 and 700 nm (emission range of the NR668 in the oil phase) and normalized by the Min-Max normalization technique (divides each spectra with its Ymax - Ymin range).

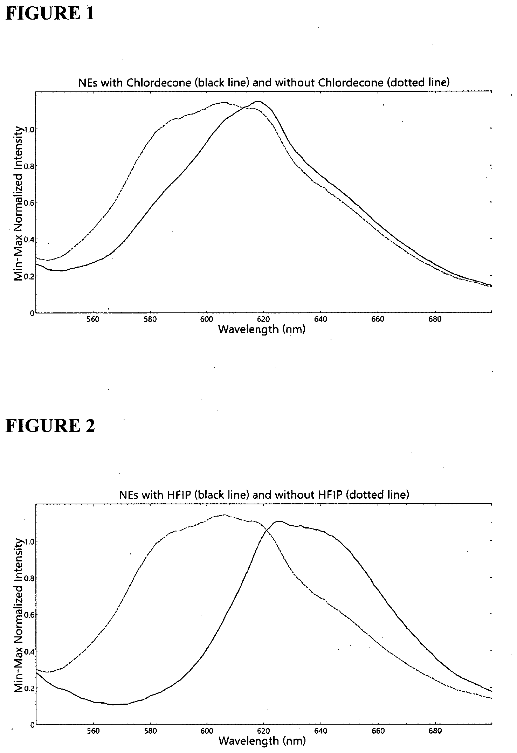

Figure 1:NEs with Chlordecone (blue) and without Chlordecone (Red)

Figure 2:NEs with HFIP(blue) and without HFIP(Red)

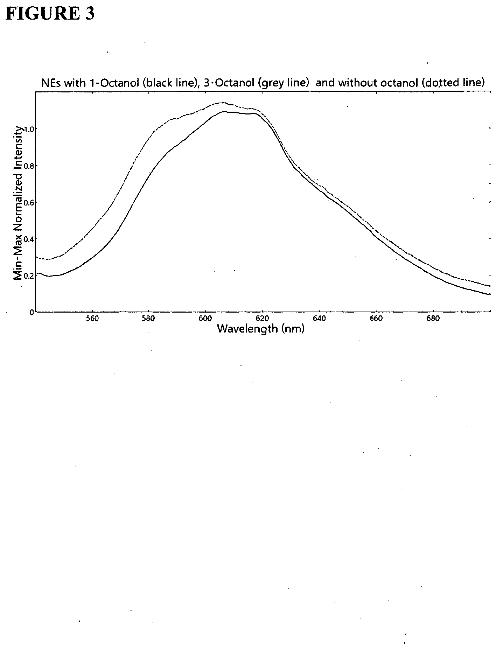

Figure 3:NEs with 1-octanol (blue), 3-Octanol (green) and without octanol (Red)

For the 4 different molecules, a variation of the fluorophore-loaded NEs spectrum could be observed. In the case of Chlordecone (Figure 1), the fluorescence distribution shifted from a distribution between 560 and 620 nm to a sharper distribution around 620 nm. For HFIP (Figure 2), the shift was even more pronounced with a distribution between 625 and 660 nm in the presence of the pollutant. For 1-Octanol and 3-Octanol (Figure 3), the results were very similar, with a slight shift to higher wavelength between 610 and 620 nm in the presence of pollutants.

Chlordecone was chosen as a model for the following experiments.

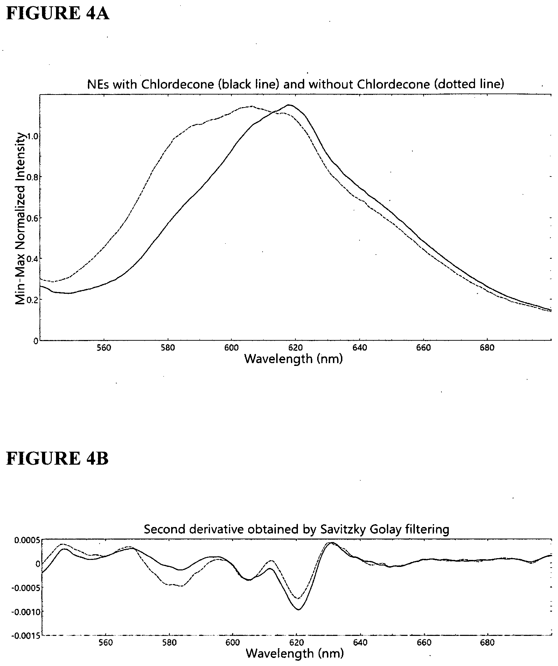

To perform a better characterization of the fluorescence shift, a signal treatment was performed as in the following publication (Watanabe et al, 2018, Solvatochromic Modeling of Laurdan for Multiple Polarity Analysis of Dihydrosphingomyelin Bilayer; Calabrese et al, 2015, Deconvolution procedure of the UV-vis spectra. A powerful tool for the estimation of the binding of a model drug to specific solubilization loci of bio-compatible aqueous surfactant-forming micelle). Savitzky-Golay filter of second derivative order is used to identify multiple peaks present in the spectral signal via identification of the second derivative minimum (Figure 4). Two main peaks could be identified in the spectral signal of both fluorophore-loaded NEs with and without Chlordecone, at wavelength of 584 nm and 620 nm.

Figure 4B: Spectral signal treatment by Savitzky-Golay Filter (Derivative Order 2, Polynomial Order 3, Window 31)

These peaks at variable wavelengths may correspond to different excited states of the NR668 and be favored by the presence of an increasing amount of analyte, herein Chlordecone.

We assessed this hypothesis by measuring fluorescence emission spectra of fluorophore-loaded NEs in the presence of a range of Chlordecone concentration. We worked with concentrations ranging from 25 µM of Chlordecone in Borate Buffer which is close to the limit of solubility to 0.1 µM of Chlordecone. The modification of spectral characteristics was assessed by visual interpretation of the spectra but also by calculating the ratio R of the intensity of the two identified peaks as described above: R=I_620/I_584

Or the difference X of the intensity of the two identified peaks as described above: X=I_620 - I_584

The spectral results are shown as mean spectrum of five replicates by concentration and restricted to the following concentrations (0, 0.1, 0.25, 1, 2.5, 10 and 25) for visual ease of interpretation, normalized by Min-Max normalization (Figure 5A). Result are consistent with the proposed mechanisms of shift from two different excited states of NR668 inside the NEs in the presence of an increasing quantity of analyte, herein Chlordecone.

Figure 5A): Normalized mean emission spectra of fluorophore-loaded NEs in presence of increasing concentration of Chlordecone from 0.1µM up to 25 µM. No Chlordecone dotted.

Figures 5B.1 and 5B.2: (5B1) Intensity ratio (620 nm over 584 nm) and (5B.2) Intensity difference (620 nm minus 584 nm) plotted in function of Chlordecone concentration on a logarithmic scale.

With both of the parameters, we were able to differentiate between samples with presence of Chlordecone and without for all concentrations, so we can claim of a LOD below 0.1 µM.

In addition, logarithmic regressions were performed for the R and X parameters and the chlordecone concentration to evaluate the quantification range of the test. Acceptable fits of r² > 0.99 were obtained for a concentration range of 0.4 µM up to 25 µM when using the difference X (r²=0,9914) and a concentration range of 1µM up to 25µM when using the ratio R (r²=0,9904) as shown in Figure 6.

Figures 6A and 6B: Logarithmic fit calculated for the ratio R=1620/1584 (6A) and the difference X=I620-I584 (6B).

### EXAMPLE 3: Soil Matrix Extraction Protocol/Results

Soil Matrix Extraction Protocol
- Weigh 5 g of soil into a glass tube
- Add 15 ml of an acidified water solution to pH=2 with 1M HCl using a pH meter
- Adjust the pH of the soil solution to pH=2 using a pH meter and 1M HCl
- Add 5 ml of a hexane/acetone mixture 85:15 (v/v)
- Shake using a rocking shaker for 10 minutes at 70 rpm, then centrifuge at 1200 rpm for 10 minutes. The supernatant (organic phase) is collected and stored in a new glass tube. • Add 5 ml of a Hexane/Acetone mixture 85:15 (v/v) to the aqueous phase of the first tube
- Shake using a rocking shaker for 10 min at 70 rpm, then centrifuge at 1200 rpm for 10 min. The supernatant (organic phase) is added to the previous fraction, the residue is evaporated to dryness and then taken up in 10 mL milliQ water for NES analysis

See Figure 7: Emission Spectra of River Water from Guadeloupe spiked with increasing concentration of chlordecone, obtained on Synergy H1 instrument

We present here a proof of concept of an engineered simplified rapid chlordecone test using fluorescent dye-loaded NEs able to provide a shift fluorescence spectra in response to aqueous sample containing chlordecone.

### Bibliography

1.Fluorescence Detection of the Persistent Organic Pollutant Chlordecone in Water at Environmental Concentrations. Oriane Della-Negra et al. Chem. Eur. J. 2023, 29, e202203887
2.Light-triggered release from dye-loaded fluorescent lipid nanocarriers in vitro and in vivo. R. Bouchaala et al. / Colloids and Surfaces B: Biointerfaces 156 (2017) 414-421.
3. Dye-Loaded Nanoemulsions: Biomimetic Fluorescent Nanocarriers for Bioimaging and Nanomedicine. Andrey S Klymchenko et al. Biomimetic Fluorescent Nanocarriers for Bioimaging and Nanomedicine. Advanced Healthcare Materials, 2020, pp.2001289. 10.1002/adhm.202001289. hal-03086886
4. Detection and quantification of chlordecone in contaminated soils from the French West Indies by GC-MS using the 13C10-chlordecone stable isotope as a tracer. Fabrice Martin-Laurent et al. Environ Sci Pollut Res (2014) 21:4928-4933
5. Detection of Organochlorine Pesticides in Contaminated Marine Environments via Cyclodextrin-Promoted Fluorescence Modulation. Dana J. DiScenza et al. ACS Omega 2017 2 (12), 8591-8599, DOI: 10.1021/acsomega.7b00991

## Claims

1. Method for determining the presence and/or the quantity of pollutants in a liquid sample, said determination being based on fluorescence spectra obtained from fluorophore-loaded nanoemulsions (NEs), said method comprising the following steps of:
A) contacting said sample to be tested and susceptible to contain said pollutants with fluorophores-loaded NEs;
B) recording/capturing the fluorescence emission spectra obtained with an excitation range from 520 to 536 nm, preferably at 520 nm ;
C) determining the presence and/or the quantity of pollutants by analysing the fluorescence emission spectra obtained in step B,
wherein prior to step A, said fluorophore-loaded NEs is prepared by the following steps of :
i) dissolving the fluorophores in oil and full homogenising the mixture;
ii) complementing the fluorophore-in-oil mixture with surfactant and full homogenising the mixture:
iii) adding water, and mixing.

2. The method according to claim 1, wherein said pollutants is selected from the group of organochlorine compounds.

3. The method according to claim 1 or 2, wherein said pollutant is chlordecone or derived compounds thereof.

4. The method according to one of claims 1 to 3, wherein in step i), fluorophore is dissolved in oil, preferably with loading of 1% in mass fraction, heated to 80 °C, mixed at 2 000 rpm, and vortexed until the full homogenization of the mixture.

5. The method according to one of claims 1 to 4, wherein in step ii),
- said fluorophore-in-oil mixture obtained in step i) is complemented with surfactant to a ratio oil/surfactant comprised between 40:60 and 60:40 in mass, preferably to a ratio of 55:45 in mass.

6. The method according to one of claims 1 to 5 wherein in step iii),
- the water is added to a ratio water/oil comprised between 70:30 and 30:70, preferably to a ratio of 60:40 in mass, and
- said fluorophore-in-oil mixture obtained in step ii) is mixed for a time comprised between 5 and 15 min, preferably 10 min. at room temperature.

7. The method according to one of claims 1 to 6, wherein:
- in steps i) to iii):
Oil is Labrafac^{™} CC, Fluorophore is NR668 , a lipophilic version of Nile red, the surfactant is Kolliphor^{®} ELP and the aqueous phase is Milli-Q water.

8. The method according to one of claims 1 to 7, wherein the fluorophore-loaded NEs were used at 1:25 000 dilution in a buffer at pH comprised between 8.0 and 9.0, preferably a borate buffer, at pH 8.6.

9. The method according to one of claims 1 to 8 wherein the sample to be tested is mixed with the fluorophore-loaded NEs 1:25 000 dilutions in equal volume.

10. The method according to one of claims 1 to 9, wherein the spectral emission datas were recorded between 350 nm and 775 nm on the IndiGo fluo (excitation LED at 520 nm), preferably the spectral emission is analysed between 540 and 700 nm.

11. The method according to one of claims 1 to 10, wherein the spectral characteristics was assessed by calculating the ratio R of the intensity fluorescence at two identified peaks and/or the difference X of the intensity fluorescence at said two identified peaks,

12. The method according to one of claims 1 to 10, wherein the sample to be tested is in presented in a liquid form, after extraction of the pollutants susceptible to be present from solid material, from soil or food.

13. Method for the production of fluorophore-loaded NEs comprising the steps i), ii) and iii) as defined in one of claims 1 to 8.

14. Kit comprising fluorophore-in-oil mixture obtained in step iii) according to the method of one of claims 1 to 8, wherein said fluorophore-in-oil mixture obtained in step iii) are diluted an aqueous phase, preferably in a buffer at pH comprised between 8 and 9, more preferably in a borate buffer at pH 8.6

15. Kit comprising fluorophore-loaded NEs obtained by the method of claim 13.

16. Use of a kit of claim 14 or 15 for determining the presence or the quantity of a given pollutant susceptible to be present in a liquid sample, wherein said liquid sample to be tested is in aqueous phase.
